# EUROPEAN PATENT APPLICATION

(11) **EP 2 199 980 A1**
(43) Date of publication of application: **23.06.2010**
(21) Application number: 08021327.5
(22) Date of filing: 09.12.2008
(51) Int. Cl.: G06T 7/00, G06F 19/00

(54) **Method and device for measuring the efficacy of plaque removal**

(71) Applicant: Braun GmbH, 61476 Kronberg im Taunus (DE)
(72) Inventor: Wilhelm, Joachim, 60389 Frankfurt/Main (DE); Waldmeier, Michael, 60322 Frankfurt/Main (DE); Schamberg, Stefan, 61250 Usingen (DE); Timm, Hans, 64839 Münster/Altheim (DE); Trebitz, Bernd, 35510 Butzbach (DE)

(57) **Abstract**

The invention relates to a method for measuring the efficacy of a dental care product with regard to plaque removal on the teeth of a volunteer, comprising the following steps:
a) requesting selection of a plaque index system;
b) requesting selection of a model tooth to be examined and display of the selected model tooth on the display device; and
c) input of the plaque data on the model tooth displayed on the display device according to the selected plaque index.

Steps b) and c) are repeated for all of the volunteer's teeth.

For each tooth, the percentage amount of plaque is determined, wherein the percentage amount of plaque corresponds to the area of the tooth surface covered with plaque according to the selected plaque index in relation to the total tooth surface. To perform the measurements, a display device with a memory device, a computer unit and an input stylus is provided for entering the plaque determined on the tooth of a volunteer on said display device.

## Description

### FIELD OF THE INVENTION

The invention relates to the development and production of dental care products, in particular toothbrushes.

### BACKGROUND OF THE INVENTION

In the development and production of dental care products, in particular toothbrushes and toothpaste, the efficacy of the cleaning performance of the product to be developed and produced plays a special role. Determining the efficacy of plaque removal by the dental care products is therefore a central component of the development and production process. For evaluating the efficacy of plaque removal, it is necessary to test the dental care products that have been developed in clinical trials on several test subjects, so-called volunteers, and to measure the plaque removal achieved with these dental care products.

US Patent 7,324,661 B2 describes a method and a device for automatic analysis of plaque removal. This method is based on an automatic analysis of teeth recorded by a camera. Although this method has the advantage that effective plaque removal is determined automatically for the most part by analyzing a tooth that has been photographed by using various image-processing algorithms and supplying a percentage amount of plaque on the surface of the tooth as the result of the analysis, this method has the disadvantage that this degree of automation leads to an inferior quality of the analytical results. The system proposed here must thus first subject an image that has been recorded to preprocessing to compensate for or remove different light conditions and shadows, for example, in two photographs of the same tooth. As a result of this preprocessing, some important image information is lost, but this information would be necessary for the subsequent step proposed there. With this watershed method, the image that has been recorded and preprocessed is segmented to thereby be able to determine the amount of plaque on the tooth surface. The watershed method has the disadvantage that segmentation errors of up to 15% may have to be acceptable. If it is additionally recalled that important information is lost even in the preprocessing step, this leads to an unacceptable result in determination of the efficacy of plaque removal.

### OBJECT OF THE INVENTION

The object of the present invention is to provide a method and a device for measuring the efficacy of plaque removal, which will avoid the known disadvantages at least in part.

### INVENTIVE APPROACH

According to the invention, this object is achieved with a method for measuring the efficacy of plaque removal on the teeth of a volunteer and with a device for performing this method according to the features of the independent claims.

Accordingly, a method for measuring the efficacy of a dental care product with regard to plaque removal on the teeth of a volunteer is made available, comprising at least the following steps:
a) requesting selection of plaque index system on a display device;
b) requesting selection of a model tooth on the display device and display of the selected model tooth on the display device;
c) requesting input of the plaque detected on a volunteer's tooth onto the model tooth depicted on the display device according to the selected plaque index;
d) repeating steps b) and c) for a predetermined number of teeth; and
e) determining the plaque fraction for each tooth by calculating the ratio of the surface area covered by plaque to the total dental surface, such that the surface covered with plaque is calculated accordingly from the selected plaque index.

The advantage of this method is that in comparison with known methods based on image-processing algorithms, the result is better here with regard to the precision of the plaque fraction on the dental surface of a volunteer.

According to the present invention, model teeth are stored in the system for each volunteer. The corresponding model tooth is selected from these model teeth and is displayed on the display device. This has the advantage that it is not necessary to take photographs of the volunteer's teeth and analyze them.

It has proven advantageous in step c) to produce at least one closed traverse on the surface of the model tooth, circumscribing the plaque detected on the tooth of the volunteer. Before determining the amount of plaque for one tooth in step e), the surface of the model tooth is divided into cells, and the cells in which the area of the traverse overlaps with the area of the cell by at least a predetermined amount are selected for determining the amount of plaque in step e).

The tooth surface of the model tooth may be divided into cells according to the Navy Plaque Index (which was developed as part of the Navy Periodontal Screening Examination, along with the Navy Periodontal Disease Index. It reflects the plaque control status of the patient and emphasizes plaque in the cervical portion of the tooth which is in contact with the gingiva margins. Comparison of scores over time can help guide intervention to prevent periodontal disease. See also: Grossman FD, Fedi PF Jr. Navy Periodontal Screening Examination. J Am Soc Prevent Dentistry. 1973; 3: 41-45 and Hancock EB, Wirthlin MR Jr. An evaluation of the Navy periodontal screening examination. J Periodontol. 1977; 48: 63-66) , the Modified Navy Plaque Index (see Claydon et al. "The use of planimetry to record and score the modified Navy index and other area-based plaque indices", Journal of Clinical Periodontology, 1995, 22: pages 670-673) or the Rustogi Modified Navy Plaque Index (see Rustogi et al. "Refinement of the Modified Navy Plaque Index to Increase Plaque Scoring Efficiency in Gumline and Interproximal Tooth Areas" ,The Journal of Clinical Dentistry, Vol.III, Supplement C, 1992, pages C9-C12) for example. The advantage here is that the examiner need only create an image of the plaque on the model tooth in the form of a closed traverse and need no longer be concerned about an assignment of plaque areas to individual cells in a manner that is susceptible to errors.

If the Rustogi Modified Navy Plaque Index is selected as the plaque index system, then before step c), the grid of the Rustogi Modified Navy Plaque Index may be superimposed on the model tooth, and in step c) the grid cells in which there is plaque on the volunteer's tooth for determining the amount of plaque in step e) may be selected.

Steps a) to e) are performed before using a dental care product on the volunteer's teeth and after use of the dental care product on the volunteer's teeth. Then the difference between the amounts of plaque before use of the dental care product and the amounts of plaque after use of the dental care product can be determined, and the difference serves as a measure of the efficacy of plaque removal.

In step e) the surface covered with plaque can be determined by numerical methods. In using plaque index systems based on a grid applied to the dental surface, the surfaces of the grid cells can be stored in the system even before performing the method.

It is especially advantageous if the model tooth displayed on the display device is rotated by 180° in the display. Then the model tooth is displayed for the examiner exactly as it appears to the volunteer.

The present invention also makes available a device for performing a measurement of the efficacy of a dental care product with regard to plaque removal on the teeth of a volunteer. The apparatus has a display device which has a memory device for storing model teeth of various model bites, various plaque index systems and a control unit for performing the steps of the method according to the invention. The device also has a computer unit for performing the method steps.

### BRIEF DESCRIPTION OF FIGURES

Additional features, possible applications and advantages of the invention are derived from the following description of exemplary embodiments of the invention which are illustrated in the figures.
Fig. 1 shows an embodiment of a device for performing the inventive method;
Fig. 2a shows an example of input of plaque data on a dental surface facially and lingually according to the planimetry method;
Fig. 2b shows input support for input of plaque data according to the planimetry method;
Fig. 3 shows the arrangement of the Rustogi Modified Navy Plaque Index on a dental surface facially and lingually;
Fig. 4 shows the arrangement of the Turesky Index on a dental surface facially and lingually; and
Fig. 5 shows an example of the combination of the Rustogi Modified Navy Plaque Index with the planimetry method for determining the plaque on the dental surface.

### DETAILED DESCRIPTION OF FIGURES

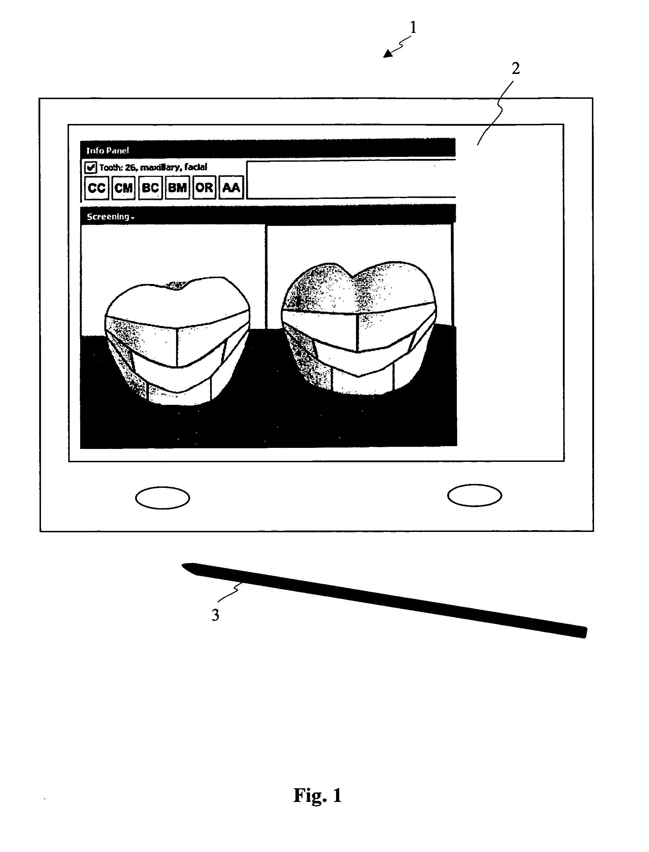

Fig. 1 shows a schematic diagram of a display and input device 1 for performing the inventive method for measuring the efficacy of plaque removal on the teeth of a volunteer. The display and input device 1 is preferably a portable display device with a touch-sensitive display device 2, e.g., a touchscreen. For operation of the display device 2, preferably an input stylus 3 is provided, because data input can be performed more accurately by using a stylus in comparison with a mouse.

The display and input device 1 has a memory device for storing teeth, i.e., dental models of various teeth. Preferably a facial view and a lingual view are saved for each of the individual teeth. In addition, different plaque index systems can be saved for performing measurements of the efficacy of plaque removal.

Determination of the efficacy of plaque removal by dental care products is a central component of the development and manufacturing process. To evaluate the efficacy of plaque removal, the dental care products that have been developed must be tested on several volunteers in experimental series and the plaque removal achieved with the dental care products must be measured.

To support the planning and implementation of experimental series, it is also advantageous to store data on volunteers and testing times in the memory device. In addition, various dental care products, e.g., toothbrushes may be stored in the memory device. During the planning of an experimental series which can be performed on the display and input device 1, various dental care products can be assigned to different volunteers. Alternatively, dental care products may also be assigned to testing sessions.

For the analysis and comparison of different experimental series that have been conducted and/or between experiments on different volunteers, it is also advantageous to store the measured data on the experiments and/or experimental series.

To ensure a high quality of the studies that are performed, the display and input device 1 is used to provide different types of access to the system to different types of users with regard to their authorization. A coordinator may design a study, obtain volunteers, organize appointments and create dental care products. An examiner, i.e., the person performing the measurements on the volunteer, can only access the teeth of one session to be tested and can store the data for performing the measurements. The examiner has no access to the information about which dental care product is being tested. A reader can analyze the study but does not have any access to the personal information on the volunteers. Finally, an administrator is provided to take care of the users of the system, but he does not have access to other data. An examiner and a coordinator do not have access to the data of a study that has not been fully completed; this is done to prevent any influence on the results of the measurements that are performed.

To ensure good clinical practice in a study, various types of users are defined:
- Coordinator: Designs the study, acquires the volunteers in the database, organizes the schedules and designs the dental care product (toothbrush and/or tooth-paste).
- Examiner: Performs the plaque determination and assesses the condition of the teeth.
- Reader: Can analyze a study.
- Administrator: Administers the users.

Essentially the method proceeds in three phases:
- a first phase, the so-called pre-visit phase,
- a second phase in which the dental care product is used by the volunteers, and
- a post-visit phase.

In the pre-visit phase the plaque distribution on the dental surfaces of the volunteer both facially and lingually is determined. In a first step, the coordinator is therefore instructed to make a selection from the saved plaque index systems. The selected plaque index system is then used to determine the plaque distribution on all of the volunteer's teeth.

In a second step the examiner is instructed to select the model teeth to be examined. On display device 2, the facial and lingual surfaces of the selected model tooth are then displayed. Since the examiner is leaning over the volunteer while determining the plaque data, the display of the model tooth on the display device 2 is rotated by 180°.

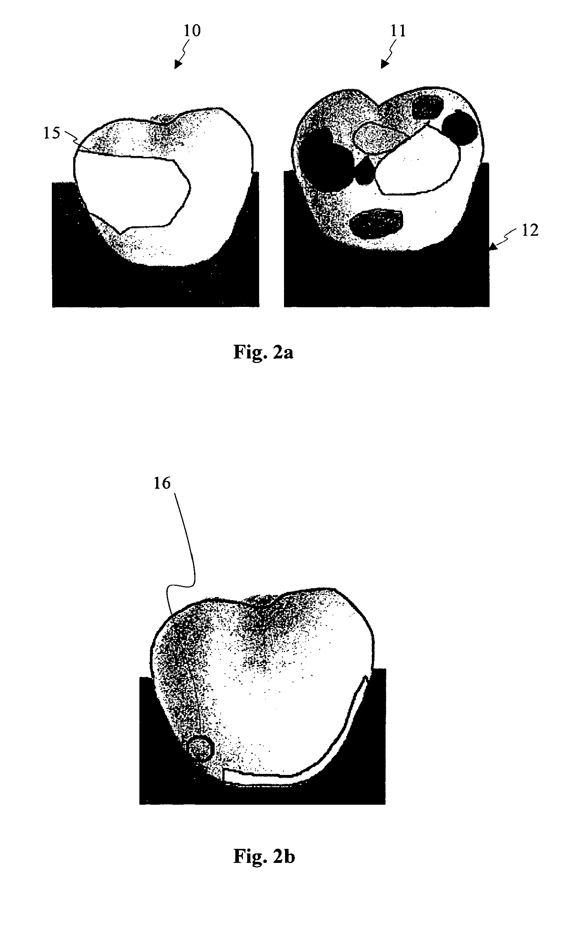

In a third step, the examiner is then instructed to enter the plaque determined on the dental surface of the volunteer on the model tooth displayed on the display device 2 according to the selected plaque index. Input of the plaque data on display device 2 advantageously takes place by using input stylus 3. With respect to Figs. 2 through 4, various methods for entering the plaque on display device 2 are described further below.

These three steps are performed for each tooth of a person's dentition. If a tooth whose input the system has requested is missing, the examiner can "remove" this tooth from the model of the teeth and the system then proceeds with the next tooth.

After the system has requested input of the plaque with respect to all of the volunteer's teeth, then in a fourth step the percentage amount of plaque is ascertained for each of the volunteer's teeth. The percentage amount of plaque is obtained from the ratio of the area with plaque to the total surface area of the tooth (facial and lingual).

These four steps are then also performed in the post-visit phase following the second phase. Thus, before and after a dental care product is used by a volunteer, the percentage amount of plaque on the dental surfaces is measured so that these two measurement results can be compared and the efficacy of plaque removal can be measured.

For example, the planimetry method, the Navy Plaque Index, the Modified Navy Plaque Index, the Rustogi Modified Navy Plaque Index, the Turesky method and combinations thereof are available as plaque index systems. Additional plaque index systems can be stored in the system at any time.

Input of plaque data for a selected model tooth according to the planimetry method on the display device 2 is explained on the basis of Fig. 2a. For the selected model tooth, both the lingual surface 10 and the facial surface 11 are displayed on display device 2. The examiner then examines the corresponding tooth of the volunteer and, using the input stylus 3, draws a closed traverse 15 on the corresponding surface on the display device 2 according to the plaque on the dental surface. The closed traverse and/or the area formed thereby may be colored with a color different from the tooth surface. The color may depend on the type of plaque. For example, a different color may be provided for thin, thick, new and old plaque, respectively, as illustrated on the facial surface 11 in Fig. 2a.

After drawing the traverses, the area formed by this traverse is automatically calculated to be able to determine the ratio of plaque area to total tooth surface area. Numerical methods may be used for the calculation of the traverse area.

An important advantage of the invention is an input (by using a stylus 3) and calculation of the planimetry. In comparison with the RMNPI and Turesky methods existing in the past, the actual percentage amount of plaque is calculated in relation to the total area of the tooth.

Fig. 2b illustrates one possibility for supporting the input of a traverse on the dental surface on display device 2. In the area of the tip of the stylus, a circle that can be moved using the input stylus 3 may be displayed. The circle offers the possibility of getting an orientation and/or moving more accurately at the gingival margin.

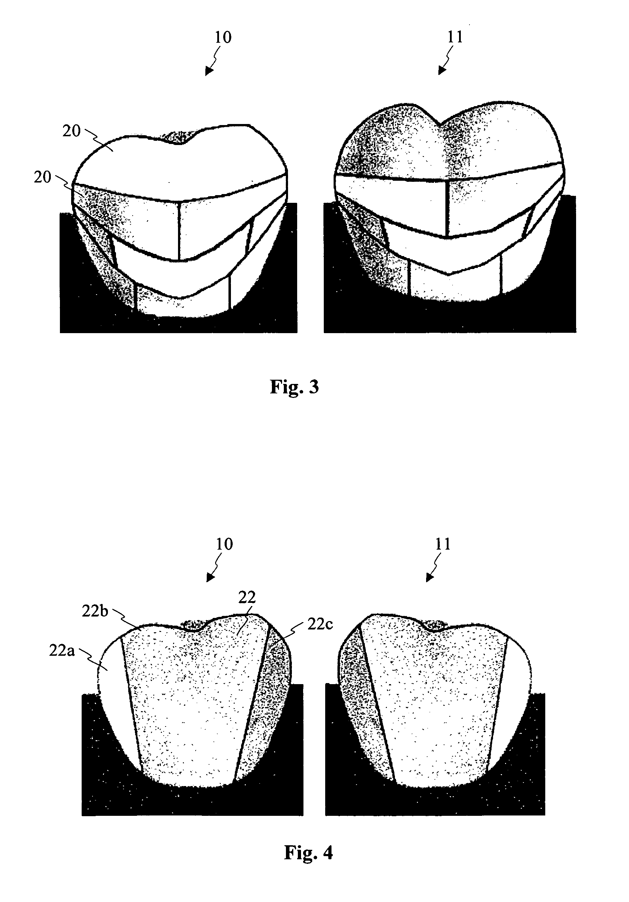

On the basis of Fig. 3, the input of plaque is explained for a selected model tooth according to the Rustogi Modified Navy Plaque Index on display device 2. On the surface of a tooth, the Rustogi Modified Navy Plaque Index forms nine areas (so-called cells) with the help of which it is possible to indicate in which locations on the tooth surface plaque is to be found. The cells are transparently superimposed on the model tooth on the display device 2.

The examiner first localizes the plaque on the volunteer's tooth surface and assigns it to one or more cells. The examiner then selects one or more cells on the display device 2 if one cell is covered by plaque to a certain degree. Preferably a cell is selected when more than 50% of the cell area is covered with plaque. The selected cell is then automatically colored completely.

The grid according to the Rustogi Modified Navy Plaque Index can be stored in the system for each surface of each tooth. However, the grid may also be generated automatically for each tooth surface as needed. According to certain specifications, the system determines the points of intersection of the grid lines based on the contour lines on the tooth surface and draws the edges of the cells through these points of intersection according to rules determined in advance. The advantage of automatic generation of the grid is that individual teeth which can be preselected by the system can be adapted by the examiner, e.g., when one tooth of a volunteer has an anomaly. After adjusting the tooth, the system recalculates the grid for the Rustogi Modified Navy Plaque Index for the corresponding tooth.

Fig. 4 shows the input of plaque data for a tooth based on a modified Turesky method. According to this method, a tooth surface (facial and lingual) is subdivided into three regions 22. These three regions are superimposed on the tooth surface displayed by a display device 2.

The regions can be defined by the line angle method, i.e., the dividing lines for the regions are the contact points of the mesial area 22c with the facial area 22b and of the distal area 22a with the facial area 22b.

The examiner first localizes the plaque on the tooth surface of the volunteer. If plaque occurs in one of these regions 22, the examiner selects the corresponding region 22 and assigns a plaque index to the selected region (e.g., 0 for "no plaque" to 5 for "more than two-thirds covered with plaque"). Preferably six different plaque indexes are provided for this.

It is also possible to provide that in the case of the modified Turesky method, the system recalculates the regions and/or the edges for a tooth surface as needed.

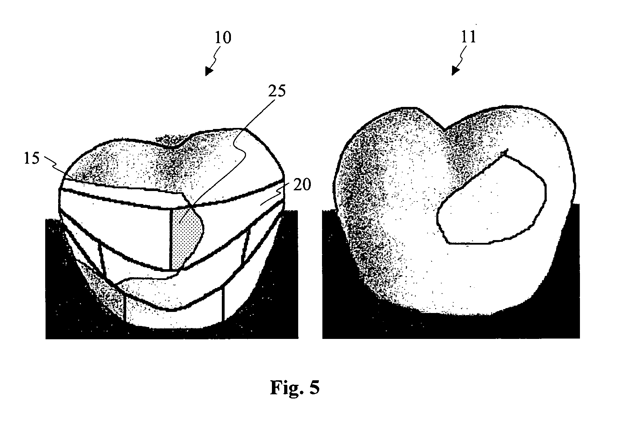

Fig. 5 shows an example for the use of two different plaque index systems. The combination of the planimetry method with the Rustogi Modified Navy Plaque Index is shown here.

First, the plaque localized on a volunteer's tooth is indicated by the examiner by drawing a closed traverse 15 on the corresponding tooth on the display device 2 according to the planimetry method, as described above with reference to Fig. 2a and Fig. 2b.

The system then automatically generates a grid according to the Rustogi Modified Navy Plaque Index over the selected tooth surface. With the help of numerical methods, the system then determines how much plaque 25 is in a cell 20 of the grid by determining the intercepting surface 25 and determining its relationship to the area of the corresponding cell 20. If the plaque assumes more than a threshold value of 50% of the respective cell area 20, the system automatically assesses this field as "cell with plaque." The cell may then be colored-in automatically with a predetermined color. The threshold value for the assessment can be adapted. The threshold value for the assessment can also be adjusted differently for different cells 20.

The combination of the planimetry method with the Rustogi Modified Navy Plaque Index has the advantage that the results for the Rustogi Modified Navy Plaque Index are qualitatively better than the results achieved with the Rustogi Modified Navy Plaque Index without prior use of the planimetry method because the examiner need not make an assignment of plaque on the tooth surface of the volunteer to a cell in the grid according to the Rustogi Modified Navy Plaque Index.

This method may also be used for the combination of the planimetry method with the Turesky method and/or with the Modified Turesky Method illustrated in Fig. 4.

With the help of the method according to the present invention, the development and production of dental care products, in particular toothbrushes and/or toothpaste can be optimized with regard to the efficacy of plaque removal.

### LIST OF REFERENCE NUMERALS

- 1: display and input device
- 2: display device
- 3: input stylus
- 10: lingual surface of the tooth
- 11: facial surface of the tooth
- 12: gingiva
- 15: traverse according to the planimetry method
- 16: circle for supporting input
- 20: cell according to the Rustogi Modified Navy Plaque Index
- 22: region according to the Modified Turesky Method
- 22a: distal area of the tooth surface
- 22b: facial area of the tooth surface
- 22c: mesial area of the tooth surface
- 25: overlap of a cell according to the Rustogi Modified Navy Plaque Index with an area according to the planimetry method.

## Claims

1. A method for measuring the efficacy of a dental care product with respect to the removal of plaque on the teeth of a volunteer, comprising the steps:
a) requesting selection of a plaque index system on a display device;
b) requesting selection of a model tooth on the display device (2) and display of the selected model tooth on the display device;
c) requesting input of the plaque data determined on a volunteer's tooth on the model tooth displayed on the display device (2) according to the selected plaque index, wherein the volunteer's tooth corresponds to the selected model tooth,
d) repeating steps b) and c) for a predetermined number of teeth; and
e) determining the percentage amount of plaque for each tooth by calculating the ratio of the area covered with plaque to the tooth surface, wherein the area covered with plaque is calculated according to the selected plaque index.

2. The method according to Claim 1, wherein in step c) at least one closed traverse (15) which outlines the plaque detected on the tooth of the volunteer is created on the surface of the model tooth, and wherein before step e)
- the surface of the model tooth is divided into cells (20, 22) and
- the cells in which the area of the traverse (15) overlaps with the area of the cell by at least a predetermined amount (25) are selected for determining the percentage amount of plaque in step e).

3. The method according to Claim 1, wherein the Rustogi Modified Navy Plaque Index is selected as the plaque index system; before step c), the grid of the Rustogi Modified Navy Plaque Index is superimposed on the model tooth; and in step c), the grid cells (20) in which plaque is found on the tooth of the volunteer are selected for determining the percentage amount of plaque in step e).

4. The method according to any one of Claims 1 to 3, wherein steps a) to e) are performed before using a dental product on the teeth of a volunteer, and the difference between the percentage amount of plaque before using the dental care product and the percentage amount of plaque after using the dental care product is determined, the difference being a measure of the efficacy of the plaque removal.

5. The method according to Claim 1, wherein at least one of these methods is used as the plaque index: the planimetry method, the Navy Plaque Index, the Modified Navy Plaque Index, the Rustogi Modified Navy Plaque Index, Turesky.

6. The method according to any one of the preceding claims, wherein the area covered with plaque is determined by numerical methods in step e).

7. The method according to any one of the preceding claims, wherein the model tooth displayed on the display device is rotated by 180° in the display.

8. The method according to any one of the preceding claims, wherein the input on the display device is accomplished by means of an input stylus (3).

9. The device for performing a measurement of the efficacy of plaque removal on the teeth of a volunteer, comprising a display device and input device (1), having a display device (2):
- a memory device for storing model teeth of different model dentitions, various plaque index systems and a control unit for executing the method according to any one of the preceding claims, and
- an input stylus (3) for entering plaque determined on the tooth of a volunteer on the display device (2).
